# EUROPÄISCHE PATENTSCHRIFT

(11) **EP 2 657 478 B1**
(45) Veröffentlichungstag und Bekanntmachung des Hinweises auf die Patenterteilung: **18.11.2015**
(21) Anmeldenummer: 12006741.8
(22) Anmeldetag: 27.09.2012
(51) Int. Cl.: F01N 13/00, F01N 11/00, F01N 3/023, F01N 3/20, F01N 13/02, F01N 3/10

(54) **Verfahren und Vorrichtung zur Überprüfung der Funktionsfähigkeit eines NO-Oxidationskatalysators**
Method and device for testing the functionality of an NO oxidation catalyst
Procédé et dispositif de vérification de la capacité de fonctionnement d'un catalyseur d'oxydation NO

(30) Priorität: 23.04.2012 DE 102012007897
(43) Veröffentlichungstag der Anmeldung: 30.10.2013
(73) Patentinhaber: MAN Truck & Bus AG, 80995 München (DE)
(72) Erfinder: Döring, Andreas, 82008 München (DE)

(56) Entgegenhaltungen:
- EP-A1- 1 936 140
- EP-B1- 1 373 693
- DE-A1-102009 000 148
- DE-B4-102004 009 615

## Beschreibung

Die Erfindung betrifft ein Verfahren zur Überprüfung der Funktionsfähigkeit eines NO-Oxidationskatalysators gemäß dem Oberbegriff des Patentanspruchs 1 sowie eine Vorrichtung zur Durchführung des Verfahrens nach Patentanspruch 16.

Brennkraftmaschinen von Kraftfahrzeugen, die mit Luftüberschuss betrieben werden, sind zur Schadstoffreduzierung mit katalytisch arbeitenden Nachbehandlungssystemen ausgerüstet, bei denen NOₓ-Speicherkatalysatoren, SCR-Katalysatoren und auch Partikelfilter eingesetzt werden, um die gesetzlich vorgeschriebenen Abgasgrenzwerte einhalten zu können. All diesen Systemen ist gemein, dass Stickstoffdioxid (NO₂) ein wichtiger Bestandteil der an den Nachbehandlungssystemen ablaufenden Reaktionen darstellt. Dieses Stickstoffdioxid wird mittels meist platinhaltiger NO-Oxidationskatalysatoren (NO = Stickstoffmonoxid) mit Hilfe der im Abgas enthaltenen Stickoxide (NOₓ) aus dem von der Brennkraftmaschine emittierten Stickstoffmonoxid gebildet:

2NO + O₂ ↔ 2NO₂ Gleichung 1

Im realen Betrieb stellt allerdings die Verschwefelung dieser NO-Oxidationskatalysatoren durch den im Kraftstoff und/oder im Motorenöl enthaltenen Schwefel ein erhebliches Problem dar. Durch die Verbrennung bildet sich durch diesen Schwefel Schwefeldioxid (SO₂), das an den NO-Oxidationskatalysatoren gemäß den nachstehenden Gleichungen zu SO₃ oxidiert wird:

S + O₂ → SO₂ Gleichung 2

2SO₂ + O₂ → SO₃ Gleichung 3

Dabei hat es sich gezeigt, dass die Menge an gebildetem SO₃ und die Menge an gebildetem NO₂ in einem direkten Zusammenhang zueinander stehen, so dass ein NO-Oxidationskatalysator, der in an sich erwünschter Weise große Mengen an NO₂ bildet, zugleich in unerwünschter Weise große Mengen an SO₃ erzeugt. Dieses SO₃ bildet mit dem metallhaltigen Katalysator-Washcoat Sulfate oder mit Wasser Schwefelsäure, die auf der Oberfläche des Katalysators physiosorbiert werden. Beides führt zu einer Abdeckung der aktiven Zentren des Katalysators und damit zu einem Rückgang von dessen Aktivität, was wiederum die Aktivität von nachgeschalteten Abgasnachbehandlungskomponenten, wie SCR-Katalysatoren oder Partikelfilter, mindert.

Aus diesem Grund ist es notwendig, den Zustand eines NO-Oxidationskatalysators bzw. dessen Oxidationsfähigkeit zu überwachen.

Zu Drei-Wege-Katalysatoren ist ein Verfahren aus der DE 10 2004 009 615 B4 bekannt, bei dem deren Sauerstoff (O₂) Einspeicherfähigkeit mit Hilfe von periodischen Schwingungen zwischen magerem und fettem Motorbetrieb und der Beobachtung der Reaktion des Katalysators mit Hilfe einer stromabwärts angebrachten Lambdasonde ermittelt wird, und zwar dergestalt, dass im Falle einer gedämpften Amplitude der Rest-O₂ - Schwingung der Katalysator noch eine O₂-Einspeicherfähigkeit besitzen soll, während er anderenfalls nicht mehr in der Lage sein soll, O₂ aufzunehmen. Dieses Verfahren kann aus zwei Gründen bei NO-Oxidationskatalysatoren nicht angewendet werden: Zum einen besitzen diese Katalysatoren keine ausgeprägte O₂-Speicherfähigkeit, und zum anderen lassen sich die üblicherweise mager betriebenen Dieselmotoren oder direkt einspritzenden Ottomotoren nicht ohne Weiteres fett betreiben. So steigen hierbei sowohl die Rußemissionen als auch die thermische Belastung der Motoren in unerwünschter Weise extrem an.

Bei sogenannten Dieseloxidationskatalysatoren, die zur Oxidation von unverbrannten Kohlenwasserstoffen und Kohlenmonoxid dienen, wird ein zweites Verfahren aus der EP 1 373 693 B2 bekannt. Hier wird periodisch die Kohlenwasserstoffoxidation, meist durch eine späte Nacheinspritzung von Kraftstoff in den Brennraum angehoben und die Exothermie der Oxidation dieser Kohlenwasserstoffe am Dieseloxidationskatalysator mit Hilfe von Thermoelementen bestimmt. Der detektierte Temperaturanstieg wird mit einem Erwartungswert, der aus der zugegebenen Kohlenwasserstoffmenge ermittelt wird, verglichen. Weichen Mess- und Erwartungswert zu stark voneinander ab, kann auf eine Schädigung des Dieseloxidationskatalysators geschlossen werden. Der Nachteil dieses Verfahrens besteht darin, dass große Mengen Kohlenwasserstoffe zugegeben werden müssen, da andernfalls, aufgrund der großen thermischen Massen und des dadurch bedingten geringen Temperaturanstiegs, keine Reaktion des Systems beobachtet werden kann. Dies führt zu einer deutlichen Verschlechterung des Wirkungsgrades der Brennmaschine und damit zu einem Verbrauchsanstieg. Ein weiterer Nachteil ist in der hohen thermischen Belastung des Katalysators zu sehen, der bei den NO-Oxidationskatalysatoren, wie bereits oben beschrieben, zu einer Schädigung desselben führen kann. Speziell bei in Fahrzeugen verbauten Brennkraftmaschinen ergibt sich aufgrund stark wechselnder Umgebungsbedingungen und der dadurch bedingt schwankenden Temperaturverluste der Abgasanlage zudem das Problem, dass die Detektion der Temperatur einem großen Messfehler unterliegen kann.

Aus der EP 1 936 140 A1 ist bereits ein Verfahren zur Überwachung eines Abgasnachbehandlungssystems einer Brennkraftmaschine bekannt, bei dem stromaufwärts im Abgasstrom vor dem Abgasnachbehandlungssystems eine erste Lambda-Sonde zur Erfassung des Luftverhältnisses angeordnet wird und bei dem weiter stromabwärts des Abgasnachbehandlungssystems eine zweite Lambda-Sonde zur Erfassung des Luftverhältnisses angeordnet wird. Zur Überwachung der Funktionsfähigkeit des Abgasnachbehandlungssystems wird die Brennkraftmaschine in einen Betrieb überführt, in dem die aus den Zylindern abgeführten Abgase eine derart hohe Konzentration an unverbrannten Kohlenwasserstoffen aufweisen, dass die erste Sonde fehlerhaft arbeitet, und zwar dergestalt, dass diese erste Sonde ein, im Vergleich zu dem tatsächlich im Abgas vorliegenden Luftverhältnis, höheres Luftverhältnis anzeigt, wobei von einer Funktionsuntüchtigkeit des Abgasnachbehandlungssystems ausgegangen wird, falls die beiden Luftverhältnisse im Wesentlichen gleich groß sind.

Der Erfindung liegt die Aufgabe zugrunde, ein Verfahren zur Überprüfung der Funktionsfähigkeit eines NO-Oxidationskatalysators zu schaffen, der in der Abgasleitung einer mit Luftüberschuss betriebenen Brennkraftmaschine eingesetzt ist, das auf einfache Weise mit hoher Funktionssicherheit auch bei niedrigen Temperaturen des Abgasstroms durchgeführt werden kann.

Diese Aufgabe wird mit den Merkmalen des Patentanspruchs 1 gelöst. Bevorzugte Weiterbildungen des erfindungsgemäßen Verfahrens sind in den Unteransprüchen 2 bis 15 offenbart.

Das Verfahren gemäß Patentanspruch 1 sieht vor, dass in dem Abgasstrom, der dem NO-Oxidationskatalysator zugeführt wird, eine Änderung der Konzentration eines Reduktionsmittels vorgenommen wird, und dass die daraus resultierende Änderung der NOₓ-Konzentration im Abgasstrom innerhalb des NO-Oxidationskatalysators und/oder stromabwärts nach dem NO-Oxidationskatalysator ermittelt und zur Überprüfung dessen Funktionsfähigkeit ausgewertet wird. Dabei wird vorzugsweise eine definierte Menge Reduktionsmittel stromaufwärts vor dem NO-Oxidationskatalysator zugegeben und/oder erzeugt. Es besteht aber auch die Möglichkeit, den Betrieb der Brennkraftmaschine so zu verändern, dass sich in deren Abgasstrom eine Änderung der Reduktionsmittelkonzentration ergibt. Im NO-Oxidationskatalysator wird das Reduktionsmittel mittels des im Abgasstrom enthaltenen NOₓ oxidiert, so dass sich innerhalb und/oder stromabwärts nach dem NO-Oxidationskatalysator eine Änderung der NOₓ-Konzentration ergibt. Eine stromaufwärts vor dem NO-Oxidationskatalysator vorgenommene Änderung der Konzentration des im Abgas enthaltenen Reduktionsmittels muss bei ordnungsgemäß funktionierendem NO-Oxidationskatalysator innerhalb und/oder stromabwärts nach dem NO-Oxidationskatalysator eine ausreichend große Änderung der NOₓ-Konzentration geben. Ist diese Änderung der NOₓ-Konzentration zu gering, kann auf eine mangelhafte Funktion des NO-Oxidationskatalysators geschlossen werden. Eine mangelhafte oder verschlechterte Funktionsfähigkeit des NO-Oxidationskatalysators kann dann beispielsweise durch eine entsprechende Fehlermeldung angezeigt werden.

Die Messung der NOₓ-Konzentration im und/oder stromabwärts nach dem NO-Oxidationskatalysator kann auf einfache Weise mittels eines NOₓ-Sensors erfolgen, der die NOₓ-Konzentration als NOₓ-Istwerte vor und nach der Änderung der Konzentration des Reduktionsmittels misst. Die Differenz der beiden NOₓ-Istwerte ist ein Maß für die Oxidationsfähigkeit des NO-Oxidationskatalysators, wenn außer der Änderung des Reduktionsmittels keine sonstigen System-Parameter geändert wurden. Es besteht aber auch die Möglichkeit, dass für unterschiedliche sonstige System-Parameter in einem Kennfeld zugehörige NOₓ-Sollwerte abgespeichert werden, die dann nach einer Änderung der Konzentration des Reduktionsmittels mit den gemessenen NOₓ-Istwerten verglichen und ausgewertet werden.

Zur Ermittlung der NOₓ-Konzentration stromaufwärts vor dem NO-Oxidationskatalysator kann dort ein NOₓ-Sensor vorgesehen sein, wobei aber auch eine Ermittlung der NOₓ-Konzentration mittels entsprechender mathematischer Modelle unter Berücksichtigung des jeweiligen Betriebszustandes der Brennkraftmaschine vorgenommen werden kann.

Befindet sich die Brennkraftmaschine in einem stationären Betriebsmodus, ist es auch möglich, die Änderung stromabwärts nach dem NO-Oxidationskatalysator nach Erhöhen der Reduktionsmittelkonzentration mit Hilfe des Wertes vor Erhöhen der Reduktionsmittelkonzentration zu bestimmen und diesen Wert mit einem Soll- bzw. Erwartungswert für die NOₓ-Konzentration zu vergleichen. Eine zugegebene und/oder erzeugte Menge an Reduktionsmittel kann so vorgegeben sein, dass das Reduktionsmittel die im Abgasstrom vor dem NO-Oxidationskatalysator enthaltene Stickoxyde (NOₓ) vollständig reduziert, wenn der NO-Oxidationskatalysator ordnungsgemäß funktioniert. Sollte dann bei einer derartigen vorgegebenen Menge an Reduktionsmittel keine vollständige Reduzierung der Stickoxide festgestellt werden, bedeutet dies, dass der NO-Oxidationskatalysator nicht mehr ordnungsgemäß funktioniert. Auf eine exakte Bestimmung der NOₓ-Konzentration kann hierbei verzichtet werden und statt dessen auf eine Fallunterscheidung "noch NOₓ vorhanden" oder "kein NOₓ mehr vorhanden". Dementsprechend können sehr kostengünstige Sprung-NOₓ-Sensoren, die eine sprunghafte Änderung des Signals liefern, wenn sich der Zustand von "noch NOₓ vorhanden" zu "kein NOₓ mehr vorhanden" ändert.

Es bietet sich an, als Reduktionsmittel Kohlenwasserstoff oder Kohlenmonoxid zu verwenden, die beide durch Verstellen von Motorparametern, wie z.B. durch Verschieben der Kraftstoffeinspritzung nach spät und/oder durch Aktivieren einer späten Nacheinspritzung, ohne zusätzliche Vorrichtungen erzeugt werden können.

Diese Reduktionsmittel reagieren gemäß den folgenden Gleichungen mit den im Abgas enthaltenen Stickoxiden:

"HC" + 2 NO → N₂ + H₂O + CO₂ Gleichung 4

2 CO + 2 NO → N₂ + 2 CO₂ Gleichung 5

Der Einsatz von Kohlenmonoxid gegenüber Kohlenwasserstoffen hat den Vorteil, dass Kohlenmonoxid bei deutlich niedrigeren Temperaturen oxidiert wird, als dies bei langkettigen Kohlenwasserstoffen aus dem Kraftstoff der Fall ist. Dies führt dazu, dass beim Stand der Technik unterhalb einer bestimmten Temperatur kein Umsatz und somit auch keine Überprüfung mit Hilfe von Kohlenwasserstoffen möglich ist, da sowohl der Sollwert als auch der Istwert Null ist. Daher ist der Einsatz von Kohlenmonoxid als Reduktionsmittel insbesondere bei solchen tiefen Temperaturen von Vorteil, wie sie bei Brennkraftmaschinen auftreten, bei denen wenigstens ein Abgasturbolader zum Einsatz gelangt. Ein weiteres Problem bei der Verwendung von Kohlenwasserstoffen stellt das Verkoken der Katalysatoren durch langkettige Kohlenwasserstoffverbindungen, insbesondere bei tiefen Temperaturen, dar.

Eine bevorzugte Weiterbildung des Verfahrens sieht vor, dass das Reduktionsmittel, welches zur Überwachung der Funktionsfähigkeit des NO-Oxidationskatalysators benötigt wird, durch homogene Kompressionszündung der Brennkraftmaschine oder durch teilhomogenen Brennkraftmaschinenbetrieb erzeugt wird. Bei der homogenen Kompressionszündung (Englisch: homogenous charge compression ignition, abgekürzt HCCI, oder auch controlled auto ignition, abgekürzt CAI) zur Anhebung der Kohlenmonoxidemission in der Überprüfungsphase handelt es sich um ein Brennverfahren, bei dem der Kraftstoff möglichst homogen im Brennraum verteilt und anschließend durch die Kompression selbst gezündet wird. Dies erfolgt meist durch eine definierte Vermischung von Kraftstoff und Frischluft vor den Zylindern der Brennkraftmaschine. Ziel dieser homogenen Kompressionszündung ist es, die Verbrennung im gesamten Brennraum möglichst gleichzeitig zu initiieren. Durch dieses Brennverfahren ist man in der Lage, die NOₓ- und Rußemissionen erheblich abzusenken, während es auf der anderen Seite zu einem Anstieg der Kohlenmonoxidemissionen kommt.

Der Nachteil beim homogenen Brennverfahren besteht bei direkteinspritzenden Brennkraftmaschinen, bei denen im Normalbetrieb der Brennstoff direkt in den Brennraum eingedüst wird, darin, dass im Ansaugtrakt zusätzliche Vorrichtungen zum Einbringen von Kraftstoff vorgesehen werden müssen.

Bei der so genannten teilhomogenen Kompressionszündung wird bei direkteinspritzenden Brennkraftmaschinen der Einspritzzeitpunkt stark nach früh verschoben, so dass Kraftstoff und Luft sich erst im Brennraum vermischen. Aufgrund des frühen Einspritzzeitpunkts wird eine Zündung der Kraftstofftröpfchen vermieden und stattdessen deren Verdampfung durch eine relativ lange Verweilzeit begünstigt. Allerdings gelingt meist keine komplett homogene Vermischung von Kraftstoff und Luft, so dass man von einer teilhomogenen Kompressionszündung spricht. Dennoch kommt es auch hier zu einem starken Anstieg der Kohlenmonoxidemissionen. Gleichzeitig steigt üblicherweise die Emission von Kohlenwasserstoffen an, allerdings mit zwei erheblichen Unterschieden zu einem Verfahren, das auf einer Verschiebung des Einspritzzeitpunkts nach spät basiert. Zum einen sind die Kohlenmonoxidkonzentrationen bei der homogenen bzw. teilhomogenen Kompressionszündung erheblich höher als die Kohlenwasserstoffkonzentrationen, während sich das Verhältnis bei der Verstellung nach spät in Richtung Kohlenwasserstoffkonzentrationen verschiebt und diese üblicherweise erheblich höher sind als die Kohlenmonoxidkonzentrationen. Zum anderen sind die bei der homogenen bzw. teilhomogenen Kompressionszündung emittierten Kohlenwasserstoffe erheblich kurzkettiger, üblicherweise im Bereich von 1 bis 5 Kohlenstoffatomen, so dass eine Versottung der Oxidationskatalysatoren durch langkettige, pyrolisierte und kondensierte Kohlenwasserstoffe vermieden werden kann.

Besonders bevorzugt ist, insbesondere im teilhomogenen Brennkraftmaschinenbetrieb, ein Verfahren, bei dem der Einspritzzeitpunkt in der Überprüfungsphase nach früh, insbesondere auf wenigstens 15° Kurbelwinkel bis maximal 370° Kurbelwinkel vor dem oberen Zünd-Totpunkt, höchst bevorzugt auf einen Wert von 20° Kurbelwinkel bis 350° Kurbelwinkel vor dem oberen Zünd-Totpunkt, verschoben wird.

Alternativ oder bevorzugt zusätzlich dazu kann in der Überprüfungsphase, während des homogenen oder teilhomogenen Brennkraftmaschinenbetriebs, vorgesehen werden, eine von der Abgasseite zur Ladeluftseite der Brennkraftmaschine rückgeführte Abgasmenge so anzuheben, dass der Anteil des Abgases in der der Brennkraftmaschine zugeführten Ladeluft wenigstens 30 % und höchstens 80 % beträgt.

Weiter kann auch vorgesehen werden, dass die dem Brennraum der Brennkraftmaschine in der Überprüfungsphase zurückgeführte Abgasmenge und das Luft-/Kraftstoffverhältnis Lambda so variiert werden, dass die Brennraumtemperatur im Bereich von größer bis einschließlich 1,02 Lambda 1850K, im Bereich von 1,02 bis 0,98 Lambda 1600K und im Bereich kleiner 0,98 Lambda 1500K nicht übersteigt. Als weitere alternative oder zusätzliche Maßnahme kann vorgesehen werden, dass das Luft-/Kraftstoffverhältnis unter einen Lambdawert von 1 absinkt oder kleiner gleich 1 ist, wobei allerdings stromaufwärts des NO-Oxidationskatalysators noch NOₓ im Abgas enthalten ist. Weiter alternativ oder zusätzlich kann vorgesehen werden, dass der Einspritzdruck des Kraftstoffs in den Zylinder in der Überprüfungsphase um wenigstens 20 % und/oder auf wenigstens 1200 bar, höchstens jedoch auf 3500 bar, angehoben wird.

Das Verhältnis aus Kohlenmonoxid- und Kohlenwasserstoffkonzentration, beträgt im homogenen oder teilhomogenen Betrieb üblicherweise mindestens 2:1.

Gemäß einer weiteren bevorzugten Durchführung des Verfahrens kann alternaiv oder zusätzlich zur Erhöhung der Kohlenmonoxidemissionen vorgesehen werden, dass das Verdichtungsverhältnis in der Überprüfungsphase um wenigstens 20 %, höchstens jedoch um 75 %, und/oder nicht unter 6:1, abgesenkt wird.

Weiter können alternativ oder zusätzlich die Ventilöffnungszeiten in der Überprüfungsphase verändert werden. Diese Maßnahmen zielen zum einen darauf, den Zündverzug zu verlängern, so dass sich eine längere Homogenisierungsphase ergibt, zum anderen sollen sie einen zu starken Druckanstieg durch Absenken der Brenngeschwindigkeit erreichen. So ist es möglich, wenigstens ein Einlassventil vor Erreichen des unteren Ladungswechseltotpunkts zu schließen. Dadurch kommt es bei geschlossenem Auslassventil zu einer Verringerung des Brennraumdrucks und damit einhergehend zu einer Absenkung der Brennraumtemperatur. Auch ein sehr spätes Schließen des Einlassventils nach dem unteren Ladungswechseltotpunkt ist denkbar, wodurch bereits angesaugte Luft wieder über die Einlassventile ausgestoßen wird. Dieses Verfahren ist als Miller-Zyklus bekannt und führt aufgrund der geringeren Füllung zu niedrigeren Spitzendrücken während der Verbrennung. Eine weitere Möglichkeit besteht darin, die Ventilöffnungszeiten in der Überprüfungsphase derart zu verändern, dass wenigstens ein Auslassventil länger geöffnet bleibt, wodurch der Restgasanteil durch Rückströmen von Abgas aus dem Abgastrakt angehoben und dadurch bedingt die Brenngeschwindigkeit verringert wird. Die Ventilöffnungszeiten können in an sich bekannter Weise mit Hilfe eines variablen Ventiltriebs variiert werden.

Da SCR-Katalysatoren, ähnlich wie die platinhaltigen NO-Oxidationskatalysatoren, bei Anwesenheit von Kohlenwasserstoffen bzw. Kohlenmonoxid eine NOₓ-Reduktionsfähigkeit aufweisen, können sie in die Bestimmung der NO-Oxidationsfähigkeit mit einbezogen werden. Da beim SCR-Verfahren (selektive katalytische Reduktion) zur Regelung der Reduktionsmittelzugabe im Normalbetrieb üblicherweise ein NOₓ-Sensor stromabwärts hinter dem SCR-Katalysator eingesetzt ist, kann dieser NOₓ-Sensor auch für die Überprüfung der NO-Oxidationsfähigkeit des NO-Oxidationskatalysators verwendet werden. Der stromaufwärts vor dem SCR-Katalysator angeordnete NO-Oxidationskatalysator kann mittels des hinter dem SCR-Katalysator angeordneten NOₓ-Sensor bzgl. seiner Oxidationsfähigkeit überprüft werden. Während des Überprüfungsvorgangs wird eine Zugabe von Reduktionsmitteln unmittelbar vor dem SCR-Katalysator, insbesondere die Zugabe von Ammoniakvorläufersubstanzen, unterbrochen. Dadurch steigt die NOₓ-Konzentration am NOₓ-Sensor an. Anschließend wird die Reduktionsmittelzugabe vor dem NO-Oxidationskatalysator, wie oben beschrieben, vorgenommen und dann der NOₓ-Wert stromabwärts hinter dem SCR-Katalysator mit Hilfe des NOₓ-Sensors bestimmt und mit einem Erwartungswert verglichen. Wird eine zu große Abweichung vom Erwartungswert festgestellt, kann auf eine mangelhafte Oxidationsfähigkeit des NO-Oxidationskatalysators geschlossen werden. Der Erfindung liegt die weitere Aufgabe zugrunde, eine Vorrichtung zur Durchführung des erfindungsgemäßen Verfahrens anzugeben, die mit möglichst einfachen technischen Mitteln realisierbar ist.

Die Lösung dieser weiteren Aufgabe wird mit einer Vorrichtung erhalten, die im Patentanspruch 16 offenbart ist. Bevorzugte Weiterbildungen der erfindungsgemäßen Vorrichtung sind in den Unteransprüchen 17 bis 19 offenbart.

Gemäß Patentanspruch 17 ist zur Durchführung des erfindungsgemäßen Verfahrens eine Steuereinrichtung vorgesehen, die eine Änderung der Konzentration des im Abgasstrom enthaltenen Reduktionsmittels vor den NO-Oxidationskatalysator steuert und mittels einer Sensoreinrichtung die NOₓ-Konzentration im Abgasstrom innerhalb und/oder nach dem NO-Oxidationskatalysator misst und auswertet. Mittels der Steuereinrichtung kann durch eine Änderung der Betriebszustände der Brennkraftmaschine eine Veränderung der Konzentration des Reduktionsmittels im Abgasstrom der Brennkraftmaschine erreicht werden, wodurch sich entsprechende Änderungen der von der Sensoreinrichtung gemessenen NOₓ-Konzentration ergeben müssen, wenn eine ordnungsgemäße Funktionsfähigkeit des NO-Oxidationskatalysators gegeben ist. Werden unzulässig starke Abweichungen von vorgegebenen Sollwerten für die NOₓ-Konzentration festgestellt, kann die Steuereinrichtung eine entsprechende Fehlermeldung auslösen.

Besonders vorteilhaft ist es, einen zusätzlichen NOₓ-Sensor stromaufwärts vor dem NO-Oxidationskatalysator anzuordnen, um auch hier eine exakte Bestimmung der NOₓ-Konzentration im Abgasstrom zu ermöglichen.

Eine weitere vorteilhafte Ausgestaltung der erfindungsgemäßen Vorrichtung verwendet eine Sensoreinrichtung zur Bestimmung der NOₓ-Konzentration im Abgasstrom hinter dem SCR-Katalysator. Auf diese Weise wird der SCR-Katalysator in die Überprüfung der Oxidationsfähigkeit mit eingeschlossen.

Die Erfindung wird nachfolgend anhand von in der Zeichnung dargestellten Ausführungsbeispielen näher erläutert.

Es zeigen:
- Fig. 1: eine schematische Darstellung einer Brennkraftmaschine mit einem Abgasnachbehandlungssystem, welches einen NO-Oxidationskatalysator umfasst, und
- Fig. 2: ein Ablaufdiagramm zur Erläuterung des Verfahrens bei stationär betriebener Brennkraftmaschine.

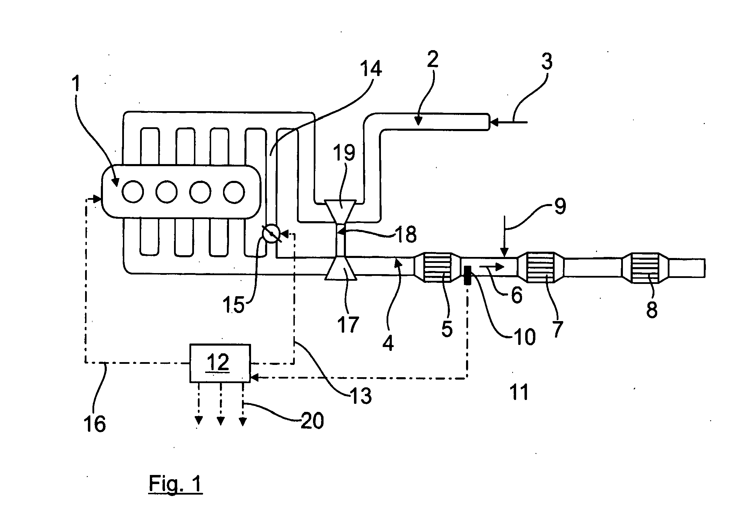

Figur 1 zeigt eine Brennkraftmaschine 1, der über eine Ladeluftleitung 2 Ladeluft 3 zugeführt wird. Von der Brennkraftmaschine 1 führt eine Abgasleitung 4 zu einem NO-Oxidationskatalysator 5, dem in Strömungsrichtung 6 ein SCR-Katalysator 7 folgt. In Strömungsrichtung 6 hinter dem SCR-Katalysator 7 ist ein Partikelfilter 8 angeordnet. In Strömungsrichtung vor dem SCR-Katalysator 7 ist mit einem Pfeil 9 eine Zudosierung eines Reduktionsmittels, beispielsweise einer wässrigen Ammoniaklösung, angedeutet. Etwas weiter stromaufwärts ist eine NOₓ-Messeinrichtung 10 in der Abgasleitung 4 angeordnet, die über eine Signalleitung 11 mit einer Steuereinrichtung 12 verbunden ist. Die Steuereinrichtung 12 ist außerdem über eine Steuerleitung 13 mit einem in einer Abgasrückführleitung 14 angeordneten Absperrelement 15 sowie über eine weitere Steuerleitung 16 zu Steuerzwecken mit der Brennkraftmaschine 1 verbunden.

In der Abgasleitung 4 ist vor dem NO-Oxidationskatalysator 5 eine Abgasturbine 17 eines Abgasturboladers 18 angeordnet, mittels der in an sich bekannter Weise ein Verdichter 19 in der Ladeluftleitung 2 zur Verdichtung der Ladeluft 3 angetrieben wird.

Zur Überprüfung der Funktionsfähigkeit, insbesondere der Oxidationsfähigkeit, des NO-Oxidationskatalysators 5, kann beispielsweise im normalen Betrieb der Brennkraftmaschine 1 die Kohlenwasserstoff - und/oder Kohlenmonoxidkonzentration im Abgas der Brennkraftmaschine 1 angehoben werden. Hierzu kann die Steuereinrichtung 12 eine späte Nacheinspritzung zur Erzeugung von Kohlenwasserstoffen, eine homogene Kompressionszündung der Brennkraftmaschine oder einen teilhomogenen Brennkraftmaschinenbetrieb vorgeben, um eine gewünschte Anhebung der Kohlenwasserstoff- und/oder Kohlenmonoxid-Emissionen bei der Brennkraftmaschine 1 herbeizuführen. Durch Änderung wenigstens eines Betriebsparameters der Brennkraftmaschine 1 wird dabei die Kohlenwasserstoff und/oder Kohlenmonoxidkonzentration stromaufwärts vor dem NO-Oxidationskatalysator 5 wenigstens auf den Wert der NOₓ-Konzentration stromaufwärts vor dem NO-Oxidationskatalysator 5 angehoben.

Um die Kohlenmonoxidemissionen in einer gewünschten Weise anheben zu können, kann mittels der Steuereinrichtung 12 beispielsweise das Absperrelement 15 so angesteuert werden, dass die Abgasrückführrate auf über 30 %, bezogen auf die der Brennkraftmaschine 1 zugeführte Ladeluftmenge, angehoben wird. Die rückgeführte Abgasmenge kann zusätzlich angehoben werden, indem in der eingangsseitigen Ladeluftleitung 2 eine hier nicht dargestellte Drosseleinrichtung zur Erhöhung der Druckdifferenz zwischen Abgas- und Ladeluft 3 vorgesehen wird.

Alternativ oder zusätzlich kann über die Steuerleitung 16 die Brennkraftmaschine 1 so gesteuert werden, dass das Luft-/Kraftstoffverhältnis Lambda beispielsweise auf unter 1,05 abgesenkt und/oder der Einspritzdruck um beispielsweise wenigstens 20 % bzw. auf mindestens 1200 bar erhöht wird. Alternativ oder zusätzlich kann auch ein Verschieben des Einspritzzeitpunktes bei der Brennkraftmaschine 1 auf beispielsweise mindestens 20° Kurbelwinkel, jedoch nicht mehr als 370° Kurbelwinkel, vor dem oberen Totpunkt (Zünd-OT) vorgesehen sein. Außerdem kann alternativ oder zusätzlich ein Absenken des Verdichtungsverhältnisses, insbesondere um mindestens 20 %, und/oder eine Veränderung der Ventilöffnungszeiten vorgesehen sein.

Durch eine derartige Veränderung von Betriebsparametern wird erreicht, dass große Mengen von Reduktionsmittel, insbesondere Kohlenmonoxid, im Abgasstrom der Brennkraftmaschine 1 generiert werden, die am NO-Oxidationskatalysator 5 mit Hilfe des im Abgasstrom enthaltenen NOₓ oxidiert werden. Die durch die Oxidation des Kohlenmonoxids bedingte Änderung der NOₓ-Konzentration im Abgasstrom stromabwärts des NO-Oxidationskatalysators 5 wird mittels der NOₓ-Messeinrichtung 10 als NOₓ-Istwert erfasst und zur Steuereinrichtung 12 übermittelt. In der Steuereinrichtung 12 kann der ermittelte NOₓ-Istwert mit einem vorgegebenen oder mit einem errechneten NOₓ-Sollwert als Erwartungswert verglichen werden. Ergibt dieser Vergleich eine zu große Abweichung des NOₓ-Istwertes vom NOₓ-Sollwert, kann daraus auf eine mangelhafte oder entsprechend verschlechterte Funktionsfähigkeit des NO-Oxidationskatalysators 5 geschlossen werden. Ist die festgestellte Abweichung zu groß, kann beispielsweise über eine weitere Steuerleitung 20 ein Fehlersignal von der Steuereinrichtung 12 abgegeben werden.

Grundsätzlich besteht auch die Möglichkeit, die NOₓ-Messeinrichtung 10 stromabwärts nach dem SCR-Katalysator 7 in der Abgasleitung 4 anzuordnen, so dass der SCR-Katalysator 7 in die Überprüfung der Funktionsfähigkeit mit eingeschlossen ist. Bei einer derartigen Anordnung der NOₓ-Messeinrichtung 10 wird während der Überprüfung der Funktionsfähigkeit des NO-Oxidationskatalysators 5 an der mit Pfeil 9 bezeichneten Stelle kein Reduktionsmittel für die selektive katalytische Reduktion zugegeben.

Anstelle der Erzeugung größerer Mengen von Reduktionsmittel stromaufwärts im Abgasstrom vor dem NO-Oxidationskatalysator 5 durch eine Änderung von Betriebsparametern, kann auch eine gezielte Zugabe von Kohlenwasserstoffen als Reduktionsmittel vor dem NO-Oxidationskatalysator 5 in die Abgasleitung 4 erfolgen.

Am Ende der Überprüfungsphase, in der die Funktionsfähigkeit des NO-Oxidationskatalysators 5 überprüft wird, wird die Brennkraftmaschine 1 wieder auf Normalbetrieb eingestellt. Die Überprüfungsphasen können dann in größeren Zeitabständen regelmäßig durchgeführt werden.

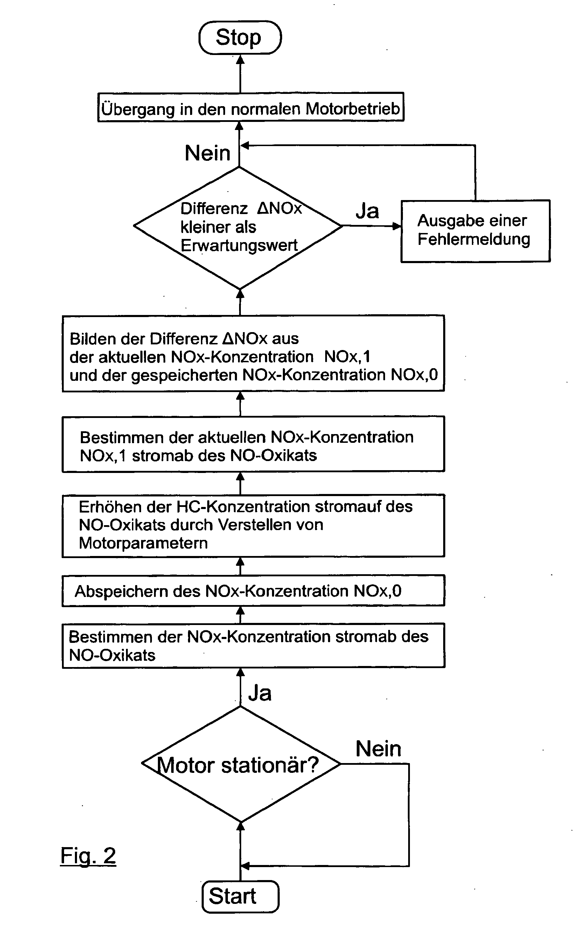

Im Ablaufdiagramm von Figur 2 sind die einzelnen Verfahrensschritte dargestellt, die bei einer Überprüfung der Funktionsfähigkeit des NO-Oxidationskatalysators 5 durchlaufen werden.

Bei dem dargestellten Verfahren wird nach dem Verfahrensstart erst geprüft, ob der Motor sich in einem stationären Betrieb befindet. Trifft dies zu, erfolgt in einem nächsten Verfahrensschritt die Bestimmung der NOₓ-Konzentration stromabwärts nach dem NO-Oxidationskatalysator 5 (kurz: NO-Oxikat). Danach wird die NOₓ-Konzentration als erster Istwert NOₓ,₀ abgespeichert. Dann wird die CO- und/oder HC-Konzentration stromaufwärts vor dem NO-Oxidationskatalysator 5 in der zuvor beschriebenen Weise, insbesondere durch das Verstellen von Betriebsparametern der Brennkraftmaschine 1, erhöht. Mittels der NOₓ-Messeinrichtung 10 von Figur 1 wird daraufhin die NOₓ-Konzentration als zweiter Istwert NO_{x,1} stromabwärts nach dem NO-Oxidationskatalysator 5 erfasst. Dieser zweite Istwert der NOₓ-Konzentration kann dann durch Differenzbildung mit dem zuvor abgespeicherten Wert NO_{x,0} verglichen werden. Für den Fall, dass die ermittelte Differenz zwischen den beiden Istwerten kleiner als ein vorgegebener Erwartungswert sein sollte, wird eine Fehlermeldung ausgegeben, die auf eine verminderte oder mangelhafte Funktion des NO-Oxidationskatalysators 5 hinweist. Ist die ermittelte Differenz jedoch größer als oder gleich wie ein vorgegebener Soll- oder Erwartungswert, so wird die Überprüfungsphase wieder verlassen und keine Fehlermeldung ausgelöst.

Für den Fall, dass die in Figur 1 vorgesehene NOₓ-Messeinrichtung 10 im Abgasstrom hinter dem SCR-Katalysator 7 angeordnet ist, wird vor dem Ermitteln der beiden Istwerte die Zugabe von Harnstoff oder einem entsprechenden, für die selektive katalytische Reduktion vorgesehenen Reduktionsmittel, gestoppt.

## Patentansprüche

1. Verfahren zur Überprüfung der Funktionsfähigkeit eines NO-Oxidationskatalysators (5), der zur Reduktion von im Abgasstrom einer mit Luftüberschuss betriebenen Brennkraftmaschine (1) enthaltenen Stickoxide (NOₓ) verwendet wird, **dadurch gekennzeichnet, dass** im Abgasstrom, der dem NO-Oxidationskatalysator (5) zugeführt wird, eine Änderung der Konzentration eines Reduktionsmittels vorgenommen wird, und dass die daraus resultierende Änderung der NOₓ-Konzentration im Abgasstrom innerhalb des NO-Oxidationskatalysators (5) und/oder stromabwärts nach den NO-Oxidationskatalysator (5) ermittelt und zur Überprüfung dessen Funktionsfähigkeit ausgewertet wird.

2. Verfahren nach Anspruch 1, **dadurch gekennzeichnet, dass** stromaufwärts vor dem NO-Oxidationskatalysator (5) eine definierte Menge eines Reduktionsmittels dem Abgasstrom der Brennkraftmaschine (1) zugeführt wird, und dass die sich daraus ergebende Änderung der NOₓ- Konzentration im Abgasstrom innerhalb und/oder stromabwärts nach dem NO-Oxidationskatalysator (5) mit einem vorgegebenen NOₓ-Sollwert verglichen wird, wobei bei einer festgestellten zu geringen NOₓ-Differenz eine nicht ausreichende Funktionsfähigkeit festgestellt wird und/oder eine Fehlermeldung erfolgt.

3. Verfahren nach einem der Ansprüche 1 oder 2, **dadurch gekennzeichnet, dass**, bevor die Menge des Reduktionsmittels geändert wird, ein NOₓ-Istwert innerhalb und/oder stromabwärts nach dem NO-Oxidationskatalysator (5) gemessen und abgespeichert wird, dass der Änderung des Reduktionsmittels ein NOₓ-Sollwert für den Abgasstrom innerhalb und/oder stromabwärts nach dem NO-Oxidationskatalysator (5) zugeordnet ist, und dass bei einer festgestellten unzulässigen Abweichung vom NOₓ-Sollwert eine nicht ausreichende Funktionsfähigkeit festgestellt wird.

4. Verfahren nach einem der vorhergehenden Ansprüche, **dadurch gekennzeichnet, dass** eine zugegebene und/oder erzeugte Menge an Reduktionsmittel so definiert und/oder vorgegeben ist, dass das Reduktionsmittel die im Abgasstrom stromaufwärts vor dem NO-Oxidationskatalysator (5) enthaltenen Stickoxide (NOₓ) vollständig reduziert.

5. Verfahren nach Anspruch 4, **dadurch gekennzeichnet, dass** die Kohlenwasserstoffkonzentration und/oder die Kohlenmonoxidkonzentration stromaufwärts vor dem NO-Oxidationskatalysator (5) höchstens so hoch ist, wie die NOₓ-Konzentration stromaufwärts vor dem NO-Oxidationskatalysator (5) und/oder dass das Verhältnis aus Kohlenmonoxid- zur Kohlenwasserstoffkonzentration mindestens 2:1 beträgt.

6. Verfahren nach einem der vorhergehenden Ansprüche, **dadurch gekennzeichnet, dass** das Reduktionsmittel, welches insbesondere Kohlenmonoxid ist, durch homogene Kompressionszündung der Brennkraftmaschine (1) oder durch teilhomogenen Brennkraftmaschinenbetrieb erzeugt wird.

7. Verfahren nach einem der vorhergehenden Ansprüche, **dadurch gekennzeichnet, dass** zur Generierung von Reduktionsmittel der Einspritzzeitpunkt des Kraftstoffs bei der Brennkraftmaschine (1), insbesondere bei einem teilhomogenen Brennkraftmaschinenbetrieb, nach früh, insbesondere auf 15° Kurbelwinkel bis maximal einschließlich 370° Kurbelwinkel vor dem oberen Zünd-Totpunkt vorteilhaft auf 20° Kurbelwinkel bis 350° Kurbelwinkel vor dem oberen Zünd-Totpunkt, verschoben wird.

8. Verfahren nach einem der vorhergehenden Ansprüche, **dadurch gekennzeichnet, dass** insbesondere bei einem teilhomogenen Brennkraftmaschinenbetrieb zur Reduktionsmittelerzeugung eine von der Abgasseite zur Ladeluftseite der Brennkraftmaschine (1) rückgeführte Abgasmenge so angehoben wird, dass der Anteil des Abgases in der der Brennkraftmaschine (1) zugeführten Ladeluft (3) wenigstens 30 % und höchstens 80 % beträt.

9. Verfahren nach einem der vorhergehenden Ansprüche, **dadurch gekennzeichnet, dass** zur Erzeugung von Reduktionsmittel die dem Brennraum der Brennkraftmaschine (1) zurückgeführte Abgasmenge und das Luft-/Kraftstoffverhältnis Lambda so variiert werden, dass die Brennraumtemperatur im Bereich größer bis einschließlich 1,02 Lambda, insbesondere im Bereich von 40 bis einschließlich 1,02 Lambda, 1850K nicht übersteigt, im Bereich von 1,02 bis einschließlich 0,98 Lambda 1600K und im Bereich kleiner 0,98 Lambda 1500K nicht übersteigt.

10. Verfahren nach einem der vorhergehenden Ansprüche, **dadurch gekennzeichnet, dass** der Einspritzdruck zur Reduktionsmittelerzeugung um wenigstens 20 % und/oder auf wenigstens 1200 bar bis maximal 3500 bar angehoben wird.

11. Verfahren nach einem der vorhergehenden Ansprüche, **dadurch gekennzeichnet, dass** zur Reduktionsmittelerzeugung das Verdichtungsverhältnis um wenigstens 20 % bis maximal 75 % und/oder nicht unter 6:1 abgesenkt wird.

12. Verfahren nach einem der vorhergehenden Ansprüche, **dadurch gekennzeichnet, dass** die Ventilöffnungszeiten bei der Reduktionsmittelerzeugung derart geändert werden, dass die Restgasmenge angehoben wird und/oder die Brennraumtemperatur der Brennkraftmaschine (1) abgesenkt wird.

13. Verfahren nach Anspruch 12, **dadurch gekennzeichnet, dass** die Ventilöffnungszeiten derart verändert werden, dass insbesondere mit Hilfe eines variablen Ventiltriebs wenigstens ein Auslassventil der Brennkraftmaschine (1) länger geöffnet bleibt und/oder wenigstens ein Einlassventil später geöffnet und/oder später geschlossen wird.

14. Verfahren nach einem der vorhergehenden Ansprüche, **dadurch gekennzeichnet, dass** ein NOₓ-Istwert aus einer NOₓ-Messung stromaufwärts vor dem NO-Oxidationskatalysator (5) und aus einer NOₓ-Messung stromabwärts nach dem NO-Oxidationskatalysator (5) ermittelt werden.

15. Verfahren nach einem der vorhergehenden Ansprüche, **dadurch gekennzeichnet, dass** das die Messung de NOₓ-Konzentration im Abgasstrom stromabwärts nach einem SCR-Katalysator (7) vorgenommen wird, der dem NO-Oxidationskatalysator (5) im Abgasstrom nachgeordnet ist, wobei während der Überprüfung der Funktionsfähigkeit des NO-Oxidationskatalysators (5) eine Zugabe von Harnstoff oder einem entsprechenden für das SCR-Verfahren erforderliche Reduktionsmittel unterbrochen wird.

16. Vorrichtung zur Durchführung eines Verfahrens zur Überprüfung der Funktionsfähigkeit eines NO-Oxidationskatalysators (5), der in der Abgasleitung (4) einer mit Luftüberschuss betriebenen Brennkraftmaschine (1) angeordnet ist, **dadurch gekennzeichnet, dass** mittels einer Steuereinrichtung (12) die Änderung der Konzentration des im Abgasstrom enthaltenen Reduktionsmittels steuerbar, und dass mittels einer im NO-Oxidationskatalysator (5) oder stromabwärts nach dem NO-Oxidationskatalysator (5) angeordneten NOₓ-Messeinrichtung (10) die NOₓ-Konzentration im Abgasstrom messbar und zur Auswertung an die Steuereinrichtung (12) übertragbar ist, wobei die Steuereinrichtung (12) zur Ausführung des Verfahrens nach einem der vorhergehenden Ansprüche ausgebildet ist.

17. Vorrichtung nach Anspruch 16, **dadurch gekennzeichnet, dass** dem NO-Oxidationskatalysator (5) ein SCR-Katalysator (7) nachgeschaltet ist, und dass die NOₓ-Messeinrichtung (10) stromabwärts nach dem SCR-Katalysator (7) angeordnet ist.

18. Vorrichtung nach einem der vorhergehenden Ansprüche 16 oder 17, **dadurch gekennzeichnet, dass** mittels der Steuereinrichtung (12) Betriebsparameter der Brennkraftmaschine (1) zur Erzeugung von Reduktionsmittel im Abgasstrom steuerbar sind.

19. Vorrichtung nach Anspruch 18, **dadurch gekennzeichnet, dass** mittels der Steuereinrichtung (12) die Abgasrückführung und/oder Ventilöffnungszeiten und/oder Einspritzzeitpunkte für den Kraftstoff und/oder die Zufuhr der Ladeluft (3) zur Brennkraftmaschine (1) zur Erzeugung von Reduktionsmittel im Abgasstrom steuerbar ist/sind.

## Claims

1. Method for testing the function capacity of an NO oxidation catalyst (5) which is used to reduce nitrous oxides (NOₓ) contained in the exhaust gas flow of an internal combustion engine (1) operated with air surplus, **characterized in that** in the exhaust gas flow which is supplied to the NO oxidation catalyst (5), a change in concentration of a reducing agent is made and that the resulting change in NOₓ concentration in the exhaust gas flow within the NO oxidation catalyst (5) and/or downstream after the NO oxidation catalyst (5) is determined and evaluated to test its function capacity.

2. Method according to Claim 1, **characterized in that** upstream before the NO oxidation catalyst (5) a defined quantity of a reducing agent is added to the exhaust gas flow from the internal combustion engine (1) and that the resulting change in NOₓ concentration in the exhaust gas flow is compared with a predefined NOₓ nominal value within and/or downstream after the NO oxidation catalyst (5), wherein, if too low an NOₓ difference is found, an inadequate function capacity is established and/or a fault message given.

3. Method according to any of Claims 1 or 2, **characterized in that** before the quantity of reducing agent is changed, an NOₓ actual value within and/or downstream after the NO oxidation catalyst (5) is measured and stored, that an NOₓ nominal value for the exhaust gas flow within and/or downstream after the NO oxidation catalyst (5) is allocated to the change in reducing agent, and that if an unacceptable deviation from the NOₓ nominal value is found, an inadequate function capacity is established.

4. Method according to any of the preceding claims, **characterized in that** a quantity of added and/or generated reducing agent is defined and/or predefined such that the reducing agent completely reduces the nitrous oxides (NOₓ) contained in the exhaust gas flow upstream before the NO oxidation catalyst (5).

5. Method according to Claim 4, **characterized in that** the hydrocarbon concentration and/or the carbon monoxide concentration upstream before the NO oxidation catalyst (5) is at most as high as the NOₓ concentration upstream before the NO oxidation catalyst (5) and/or that the ratio of carbon monoxide concentration to hydrocarbon concentration is at least 2:1.

6. Method according to any of the preceding claims, **characterized in that** reducing agent which in particular is carbon monoxide is generated by homogenous charge compression ignition of the internal combustion engine (1) or by partly homogenous internal combustion engine operation.

7. Method according to any of the preceding claims, **characterized in that** to generate reducing agent, the injection time of the fuel in the internal combustion engine (1) is advanced, in particular in a partly homogenous internal combustion engine operation, in particular to 15° crank angle to maximum inclusive 370° crank angle before the top ignition dead centre, advantageously to 20° crank angle to 350° crank angle before the top ignition dead centre.

8. Method according to any of the preceding claims, **characterized in that** in particular in a partly homogenous internal combustion engine operation, to generate reducing agent a quantity of exhaust gas recirculated from the exhaust gas side to the charge air side of the internal combustion engine (1) is raised such that the proportion of exhaust gas in the charge air (3) supplied to the internal combustion engine (1) is at least 30% and at most 80%.

9. Method according to any of the preceding claims, **characterized in that** to generate reducing agent the quantity of exhaust gas recirculated to the combustion chamber of the internal combustion engine (1) and the air/fuel ratio lambda are varied such that the combustion chamber temperature in the range above and including 1.02 lambda, in particular in the range from 40 to 1.02 lambda inclusive, does not exceed 1850K, in the range from 1.02 to 0.98 lambda, 1600K inclusive, and in the range below 0.98 lambda, 1500K.

10. Method according to any of the preceding claims, **characterized in that** the injection pressure for generating reducing agent is raised by at least 20% and/or to at least 1200 bar to maximum 3500 bar.

11. Method according to any of the preceding claims, **characterized in that** to generate reducing agent, the compression ratio is lowered by at least 20% to maximum 75% and/or not below 6:1.

12. Method according to any of the preceding claims, **characterized in that** the valve opening times on generation of reducing agent are changed such that the residual gas quantity is increased and/or the combustion chamber temperature of the internal combustion engine (1) is reduced.

13. Method according to Claim 12, **characterized in that** the valve opening times are changed such that in particular by means of variable valve drive, at least one exhaust valve of the internal combustion engine (1) remains open longer and/or at least one inlet valve is opened later and/or closed later.

14. Method according to any of the preceding claims, **characterized in that** an NOₓ actual value is determined from an NOₓ measurement upstream before the NO oxidation catalyst (5) and from an NOₓ measurement downstream after the NO oxidation catalyst (5).

15. Method according to any of the preceding claims, **characterized in that** the NOₓ concentration is measured in the exhaust gas flow downstream after an SCR catalyst (7) which is arranged after the NO oxidation catalyst (5) in the exhaust gas flow, wherein during testing of the function capacity of the NO oxidation catalyst (5), an addition of urea or a corresponding reducing agent necessary for the SCR process is interrupted.

16. Device for performance of a method for testing the function capacity of an NO oxidation catalyst (5) which is arranged in the exhaust pipe (4) of an internal combustion engine (1) operated with air surplus, **characterized in that**, by means of a control device (12), the change in concentration of the reducing agent contained in the exhaust gas flow is controllable, and that, by means of an NOₓ measurement device (10) arranged in the NO oxidation catalyst (5) or downstream after the NO oxidation catalyst (5), the NOₓ concentration in the exhaust gas flow is measurable and is transmittable to the control device (12) for evaluation, wherein the control device (12) is configured for carrying out the method according to any of the preceding claims.

17. Device according to Claim 16, **characterized in that** an SCR catalyst (7) is connected after the NO oxidation catalyst (5) and that the NOₓ measurement device (10) is arranged downstream after the SCR catalyst (7).

18. Device according to either of the preceding Claims 16 or 17, **characterized in that**, by means of the control device (12), operating parameters of the internal combustion engine (1) to generate reducing agent in the exhaust gas flow are controllable.

19. Device according to Claim 18, **characterized in that**, by means of the control device (12), the exhaust gas recirculation and/or valve opening times and/or injection times for the fuel and/or the supply of charge air (3) to the internal combustion engine (1) in order to generate reducing agent in the exhaust gas flow is/are controllable.

## Revendications

1. Procédé pour tester l'aptitude au fonctionnement d'un catalyseur d'oxydation de NO (5), qui est utilisé pour la réduction d'oxydes d'azote (NOₓ) contenus dans le flux de gaz d'échappement d'un moteur à combustion interne (1) exploité avec un excès d'air, **caractérisé en ce qu'**on réalise, dans le flux de gaz d'échappement qui est amené au catalyseur d'oxydation de NO (5), une modification de la concentration en un agent de réduction et **en ce que** la modification de la concentration en NOₓ dans le flux de gaz d'échappement qui en résulte est déterminée dans le catalyseur d'oxydation de NO (5) et/ou en aval du catalyseur d'oxydation de NO (5) et est utilisée pour tester son aptitude au fonctionnement.

2. Procédé selon la revendication 1, **caractérisé en ce qu'**on introduit, en amont du catalyseur d'oxydation de NO (5), une quantité définie d'un agent de réduction dans le flux de gaz d'échappement du moteur à combustion interne (1) et **en ce que** la modification qui en découle de la concentration en NOₓ dans le flux de gaz d'échappement dans et/ou en aval du catalyseur d'oxydation de NO (5) est comparée à une valeur de consigne prédéfinie de NOₓ, une aptitude au fonctionnement insuffisante étant constatée et/ou un message d'erreur étant émis lors de la constatation d'une différence de NOₓ insuffisante.

3. Procédé selon l'une quelconque des revendications 1 ou 2, **caractérisé en ce qu'**on mesure et enregistre, avant la modification de la quantité d'agent de réduction, une valeur instantanée de NOₓ dans et/ou en aval du catalyseur d'oxydation de NO (5), **en ce que** la modification de l'agent de réduction est associée à une valeur de consigne de NOₓ pour le flux de gaz d'échappement dans et/ou en aval du catalyseur d'oxydation de NO (5) et **en ce qu'**une aptitude au fonctionnement insuffisante est constatée lors de la constatation d'un écart non admissible de la valeur de consigne de NOₓ.

4. Procédé selon l'une quelconque des revendications précédentes, **caractérisé en ce qu'**une quantité ajoutée et/ou générée d'agent de réduction est définie et/ou prédéterminée de manière telle que l'agent de réduction réduit complètement les oxydes d'azote (NOₓ) contenus dans le flux de gaz d'échappement en amont du catalyseur d'oxydation de NO (5).

5. Procédé selon la revendication 4, **caractérisé en ce que** la concentration en hydrocarbures et/ou la concentration en monoxyde de carbone en amont du catalyseur d'oxydation de NO (5) présente un niveau maximum qui correspond à la concentration en NOₓ en amont du catalyseur d'oxydation de NO (5) et/ou **en ce que** le rapport de la concentration en monoxyde de carbone à la concentration en hydrocarbures vaut au moins 2:1.

6. Procédé selon l'une quelconque des revendications précédentes, **caractérisé en ce que** l'agent de réduction, qui est en particulier le monoxyde de carbone, est généré par allumage par compression homogène du moteur à combustion interne (1) ou par un fonctionnement partiellement homogène du moteur à combustion interne.

7. Procédé selon l'une quelconque des revendications précédentes, **caractérisé en ce que**, pour la génération de l'agent de réduction, le moment d'injection du carburant du moteur à combustion interne (1), en particulier lors d'un fonctionnement partiellement homogène du moteur à combustion interne, est déplacé vers l'avance, en particulier à un angle de vilebrequin de 15° jusqu'à au maximum un angle de vilebrequin de 370° inclus avant le point mort haut d'allumage, avantageusement à un angle de vilebrequin de 20° jusqu'à un angle de vilebrequin de 350° avant le point mort haut d'allumage.

8. Procédé selon l'une quelconque des revendications précédentes, **caractérisé en ce que**, en particulier dans le cas d'un fonctionnement partiellement homogène d'un moteur à combustion interne, pour la génération de l'agent de réduction, une quantité de gaz d'échappement recyclée du côté gaz d'échappement vers le côté air d'alimentation du moteur à combustion interne (1) est augmentée de manière telle que la proportion du gaz d'échappement dans l'air d'alimentation (3) amené vers le moteur à combustion interne (1) est d'au moins 30% et d'au plus 80%.

9. Procédé selon l'une quelconque des revendications précédentes, **caractérisé en ce que**, pour générer l'agent de réduction, la quantité de gaz d'échappement recyclée dans la chambre de combustion du moteur à combustion interne (1) et le rapport lambda air/carburant sont variés de manière telle que la température de la chambre de combustion, dans la plage supérieure à 1,02 lambda compris, en particulier dans la plage de 40 à 1,02 lambda compris, ne dépasse pas 1850 K, dans la plage de 1,02 à 0,98 lambda ne dépasse pas 1600 K et dans la plage inférieure à 0,98 lambda ne dépasse pas 1500 K.

10. Procédé selon l'une quelconque des revendications précédentes, **caractérisé en ce que** la pression d'injection pour la génération de l'agent de réduction est élevée d'au moins 20% et/ou à au moins 1200 bars jusqu'à au maximum 3500 bars.

11. Procédé selon l'une quelconque des revendications précédentes, **caractérisé en ce que**, pour la génération de l'agent de réduction, le rapport de compression est abaissé d'au moins 20% jusqu'à au maximum 75% et/ou à pas moins de 6:1.

12. Procédé selon l'une quelconque des revendications précédentes, **caractérisé en ce que** les temps d'ouverture de la soupape lors de la génération d'agent de réduction sont modifiés de manière telle que la quantité résiduelle de gaz est élevée et/ou la température de la chambre de combustion du moteur à combustion interne (1) est abaissée.

13. Procédé selon la revendication 12, **caractérisé en ce que** les temps d'ouverture de soupape sont modifiés de manière telle qu'au moins une soupape de sortie du moteur à combustion interne (1) reste ouverte plus longtemps et/ou au moins une soupape d'entrée est ouverte plus tard et/ou fermée plus tard, en particulier à l'aide d'une commande de soupape variable.

14. Procédé selon l'une quelconque des revendications précédentes, **caractérisé en ce qu'**une valeur instantanée de NOₓ est déterminée à partir d'une mesure de NOₓ en amont du catalyseur d'oxydation de NO (5) et à partir d'une mesure de NOₓ en aval du catalyseur d'oxydation de NO (5).

15. Procédé selon l'une quelconque des revendications précédentes, **caractérisé en ce que** la mesure de la concentration en NOₓ dans le flux de gaz d'échappement est réalisée en aval d'un catalyseur de RCS (7) qui est disposé dans le flux de gaz d'échappement en aval du catalyseur d'oxydation de NO (5), une addition d'urée ou d'un agent de réduction correspondant nécessaire pour le procédé RCS étant interrompue pendant le test de l'aptitude au fonctionnement du catalyseur d'oxydation de NO (5).

16. Dispositif pour réaliser un procédé pour tester l'aptitude au fonctionnement d'un catalyseur d'oxydation de NO (5), qui est disposé dans la conduite de gaz d'échappement (4) d'un moteur à combustion interne (1) exploité avec un excès d'air, **caractérisé en ce que** la modification de la concentration en agent de réduction contenu dans le flux de gaz d'échappement peut être régulée au moyen d'un dispositif de commande (12) et **en ce que** la concentration en NOₓ dans le flux de gaz d'échappement est mesurable au moyen d'un dispositif de mesure de NOₓ (10) disposé dans le catalyseur d'oxydation de NO (5) ou en aval du catalyseur d'oxydation de NO (5) et peut être transmise au dispositif de commande (12) pour l'évaluation, le dispositif de commande (12) étant conçu pour réaliser le procédé selon l'une quelconque des revendications précédentes.

17. Dispositif selon la revendication 16, **caractérisé en ce qu'**un catalyseur RCS (7) est disposé en aval du catalyseur d'oxydation de NO (5) et **en ce que** le dispositif de mesure de NOₓ (10) est disposé en aval du catalyseur RCS (7).

18. Dispositif selon l'une quelconque des revendications précédentes 16 ou 17, **caractérisé en ce que** des paramètres de fonctionnement du moteur à combustion interne (1) pour générer l'agent de réduction dans le flux de gaz d'échappement peuvent être régulés au moyen du dispositif de commande (12).

19. Dispositif selon la revendication 18, **caractérisé en ce que** le recyclage du gaz d'échappement et/ou les temps d'ouverture de soupape et/ou les moments d'injection du carburant et/ou l'alimentation en air d'alimentation (3) vers le moteur à combustion interne (1) pour générer l'agent de réduction dans le flux de gaz d'échappement peuvent être régulés au moyen du dispositif de commande (12).
